# EUROPEAN PATENT APPLICATION

(11) **EP 1 460 563 A1**
(43) Date of publication of application: **22.09.2004**
(21) Application number: 03701002.2
(22) Date of filing: 06.01.2003
(51) Int. Cl.: G06F 17/30

(54) **KNOWLEDGE SEARCH APPARATUS, KNOWLEDGE SEARCH METHOD, PROGRAM, AND RECORDING MEDIUM**

(30) Priority: 28.12.2001 JP 2001401979
(71) Applicant: Celestar Lexico-Sciences, Inc., Chiba-shi, Chiba 261-8501 (JP)
(72) Inventor: HORAI, Hisayuki, Celestar Lexico-Sciences, Inc., Chiba-shi, Chiba 261-8501 (JP); NITTA, Kiyoshi, Celestar Lexico-Sciences, Inc., Chiba-shi, Chiba 261-8501 (JP); DOI, Hirofumi, Celestar Lexico-Sciences, Inc., Chiba-shi, Chiba 261-8501 (JP)
(74) Representative: HOFFMANN - EITLE
(86) International application number: PCT/JP2003/000010
(87) International publication number: WO 2003/058499

(57) **Abstract**

A user designates two targets a relation of which the user is to search as a search start target and a search end target, respectively. The user also designates various search conditions. A binary relation database that stores binary relational information extracted, in advance, from various types of biomolecular databases (e.g., databases that store sequence information on DNAs and the like, structural information on proteins and the like, etc), compound databases (e.g., databases that store characteristics of organic compounds and the like), and literature databases (e.g., databases that store technical literatures, papers, and the like) is accessed, and knowledge between the search start target and the search end target is automatically searched.

## Description

### TECHNICAL FIELD

The present invention relates to a knowledge search apparatus, a knowledge search method, a program, and a recording medium. More specifically, the present invention relates to a knowledge search apparatus, a knowledge search method, a program, and a recording medium capable of efficiently searching for knowledge based on a binary relation.

### BACKGROUND ART

In the field of bioinformatics, large-sized biomolecular databases about DNAs, proteins, and the like have been recently constructed, and various pieces of biological information are registered in the databases as annotation information. Large-sized compound databases about organic compounds and the like have been constructed, and various pieces of compound characteristic and structural information are registered in the databases as annotation information. In addition, large-sized literature databases about scientific papers and technical literatures have been constructed, and an environment in which the databases are accessible through the Internet or the like has been provided. Researchers and the others access these biomolecular databases, compound databases, literature databases, and the like, and search for known biological knowledge.

However, this conventional search system has the following fundamental, structural disadvantage. If one researcher is to discover unknown biological knowledge from the biomolecular databases, compound databases, literature databases, and the like, the researcher needs to access a plurality of databases and does a search while manually tracing registered biological knowledge.

This disadvantage will be explained more specifically.

If a researcher is to study an unknown relation (e.g., an unknown interaction) between two matters (e.g., a protein A and a protein B), then the researcher or searcher searches for the two matters using the known databases such as technical literature databases and DNA sequence databases, and estimates the relation between the matters based on search results.

Nevertheless, in order to carry out an operation for manually comparing the search results and estimating the relation, it is necessary for a user to read the search results and understand technical contents of the search results. Therefore, the search operation requires lots of labor and time, and also requires searcher's skill in database operation. In addition, there is a probability that the user overlooks or misunderstands the technical contents. Naturally, therefore, there is a limit to search accuracy. The conventional system and the like have thus many disadvantages. As a result, the conventional system is inferior in convenience and utilization efficiency for both the user and a manager of the system.

The conventional art and the problems solved by the present invention explained above are applied not only to the search system in the bioinformatics field but also to all other systems for searching for information accumulated in databases.

It is, therefore, an object of the present invention to provide a knowledge search apparatus, a knowledge search method, a program, and a recording medium capable of efficiently searching for knowledge based on a binary relation.

### DISCLOSURE OF THE INVENTION

The knowledge search apparatus according to one aspect of the present invention includes: a binary relational information storage unit that stores binary relational information while making a relational source target, a relational destination target, and a relation between the relational source target and the relational destination target correspond to one another; a search condition setting unit that allows a user to set a search start target and a search end target; and a search unit that searches for all of or one of the targets and the relation for the search start target and the search end target from the binary relational information stored in the binary relational information storage unit; wherein knowledge between the search start condition and the search end condition is automatically searched.

According to this apparatus, the binary relational information is stored while making the relational source target, the relational destination target, and the relation between the relational source target and the relational destination target correspond to one another, the user is allowed to set the search start target and the search end target, and all of or one of the targets and the relation between the search start target and the search end target are searched from the stored binary relational information stored. Therefore, it is possible to automatically search for the knowledge the search start condition and the search end condition. The search for the information from literatures and the like that is conventionally made manually can be thereby executed by the computer. Therefore, it is possible to promptly, easily, and completely search for information on the information stored in a plurality of large-sized databases.

The knowledge search apparatus according to another aspect of the present invention further includes: a binary relational information creation unit that creates the binary relational information based on information on the targets and the relation expressed by a monadic relation or a polynomial relation of three terms or more.

According to this apparatus, the binary relational information is created based on the information on the targets and the relation each expressed by a monadic relation or complex polynomial relation of three terms or more. Therefore, it is possible to considerably improve search efficiency by replacing each monadic relation or complex polynomial relation by the binary relation.

The knowledge search apparatus according to still another aspect of the present invention, wherein the search unit further includes: a forward search unit that searches for the binary relational information stored in the binary relational information storage unit based on the relational source target.

This shows one example of the binary relational search more specifically. According to this apparatus, the stored binary relational information is searched based on the relational source target. Therefore, it is possible to conduct a search while paying attention to the directivity the binary relation, and a search tracing the target direction in the forward direction.

The knowledge search apparatus according to still another aspect of the present invention, wherein the search unit further includes: a backward search unit that searches for the binary relational information stored in the binary relational information storage unit based on the relational destination target.

This shows one example of the binary relational search more specifically. According to this apparatus, the stored binary relational information is searched based on the relational destination target. Therefore, it is possible to conduct a search while paying attention to the directivity of the binary relation, and a search tracing the direction of the target in the backward direction.

The knowledge search apparatus according to still another aspect of the present invention, wherein the search unit further includes: a target queue storage unit that sequentially stores the search targets in a queue; a breadth-first search unit that searches for the binary relational information stored in the binary relational information storage unit in an order of storing the searched targets in the queue.

This shows one example of the binary relational search more specifically. According to this apparatus, the searched targets are sequentially stored in the queue, and the stored binary relational information is searched in the order of storing the searched target in the queue. Therefore, it is possible to efficiently search for the targets in a wide range.

The knowledge search apparatus according to still another aspect of the present invention wherein the search unit further includes: a target overlap elimination unit that excludes overlap of the targets stored in the queue, wherein the breadth-first search unit conducts a search for the queue from which the overlap of the targets is eliminated by the target overlap elimination unit.

This shows one example of the breadth-first search more specifically. According to this apparatus, the overlap of the targets stored in the queue is eliminated, and the breadth-first search unit searches for the queue from which the overlap of the targets is eliminated. Therefore, it is possible to avoid the infinite loop caused by conducting a search for the same target again.

The knowledge search apparatus according to still another aspect of the present invention, further includes: a condition setting unit that allows the user to set the conditions for all of or one of the targets and the relation to be searched, wherein the search unit further includes: a condition search unit that searches the binary relational information stored in the binary relational information storage unit by refining the binary relational information based on the conditions set by the condition setting unit.

This shows one example of the binary relational search more specifically. According to this apparatus, the user is allowed to set conditions for all of or one of the targets and relation to be searched, and the stored binary relational information is searched while refining the binary relational information based on the set conditions. Therefore, it is possible to efficiently search for information on a desired target.

The knowledge search apparatus according to still another aspect of the present invention, further includes: a synonym storage unit that stores one of the targets and the relation searched and a corresponding synonym while making the synonym correspond to one of the targets and the relation, wherein the search unit further includes: a synonym search unit that searches the synonym corresponding to one of the targets and the relation searched.

This shows one example of the binary relational search more specifically. According to this apparatus, one of the targets and the relation and the corresponding synonym are stored while making them correspond to each other, and the synonym corresponding to one of the searched targets and relation is searched. Therefore, it is possible to expand the search range up to synonyms and improve knowledge search accuracy.

The knowledge search apparatus according to still another aspect of the present invention, further includes: an ontology storage unit that stores one of the targets and the relation searched and a corresponding hierarchical concept item the hierarchical concept item correspond to one of the targets and the relation, wherein the search unit further includes: an ontology search unit that searches for all of or one of the targets and the relation included in the hierarchical concept item corresponding to one of the targets and the relation searched.

This shows one example of the binary relational search more specifically. According to this apparatus, one of the targets and the relation and the corresponding hierarchical concept item are stored while making them correspond to each other, and all of or one of the targets and the relation included in the hierarchical concept item corresponding to one of the searched targets and relation is searched. Therefore, it is possible to expand the search range up to a generic concept or a subordinate concept and further improve knowledge search accuracy.

The knowledge search apparatus according to still another aspect of the present invention, further includes: a step-execution unit that step-executes a binary relation search by the search unit based on at leas one of the number of searches done by the search unit, the number of targets searches, and hierarchies of the searches.

This shows one example of the binary relational search more specifically. According to this apparatus, the binary relational information is step-executed based on at least one of the number of searches, the number of searched targets, and hierarchies of searches. Therefore, it is possible to execute the processings while the user check the processing results to some extent and selectively conduct a search in a necessary range.

The knowledge search method according to one aspect of the present invention includes: a binary relational information storage step that stores binary relational information while making a relational source target, a relational destination target, and a relation between the relational source target and the relational destination target correspond to one another; a search condition setting step that allows a user to set a search start target and a search end target; and a search step that searches for all of or one of the targets and the relation for the search start target and the search end target from the binary relational information stored in the binary relational information storage step; wherein knowledge between the search start condition and the search end condition is automatically searched.

According to this method, the binary relational information is stored while making the relational source target, the relational destination target, and the relation between the relational source target and the relational destination target correspond to one another, the user is allowed to set the search start target and the search end target, and all of or one of the targets and the relation between the search start target and the search end target are searched from the stored binary relational information stored. Therefore, it is possible to automatically search for the knowledge the search start condition and the search end condition. The search for the information from literatures and the like that is conventionally made manually can be thereby executed by the computer. Therefore, it is possible to promptly, easily, and completely search for information on the information stored in a plurality of large-sized databases.

The knowledge search method according to another aspect of the present invention further includes: a binary relational information creation step that creates the binary relational information based on information on the targets and the relation expressed by a monadic relation or a polynomial relation of three terms or more.

According to this method, the binary relational information is created based on the information on the targets and the relation each expressed by a monadic relation or complex polynomial relation of three terms or more. Therefore, it is possible to considerably improve search efficiency by replacing each monadic relation or complex polynomial relation by the binary relation.

The knowledge search method according to still another aspect of the present invention, wherein the search step further includes: a forward search step that searches for the binary relational information stored in the binary relational information storage step based on the relational source target.

This shows one example of the binary relational search more specifically. According to this method, the stored binary relational information is searched based on the relational source target. Therefore, it is possible to conduct a search while paying attention to the directivity the binary relation, and a search tracing the target direction in the forward direction.

The knowledge search method according to still another aspect of the present invention, wherein the search step further includes: a backward search step that searches for the binary relational information stored in the binary relational information storage step based on the relational destination target.

This shows one example of the binary relational search more specifically. According to this method, the stored binary relational information is searched based on the relational destination target. Therefore, it is possible to conduct a search while paying attention to the directivity of the binary relation, and a search tracing the direction of the target in the backward direction.

The knowledge search method according to still another aspect of the present invention, wherein the search step further includes: a target queue storage step that sequentially stores the search targets in a queue; a breadth-first search step that searches for the binary relational information stored in the binary relational information storage step in an order of storing the searched targets in the queue.

This shows one example of the binary relational search more specifically. According to this method, the searched targets are sequentially stored in the queue, and the stored binary relational information is searched in the order of storing the searched target in the queue. Therefore, it is possible to efficiently search for the targets in a wide range.

The knowledge search method according to still another aspect of the present invention wherein the search step further includes: a target overlap elimination step that excludes overlap of the targets stored in the queue, wherein the breadth-first search step conducts a search for the queue from which the overlap of the targets is eliminated by the target overlap elimination step.

This shows one example of the breadth-first search more specifically. According to this method, the overlap of the targets stored in the queue is eliminated, and the breadth-first search step searches for the queue from which the overlap of the targets is eliminated. Therefore, it is possible to avoid the infinite loop caused by conducting a search for the same target again.

The knowledge search method according to still another aspect of the present invention, further includes: a condition setting step that allows the user to set the conditions for all of or one of the targets and the relation to be searched, wherein the search step further includes: a condition search step that searches the binary relational information stored in the binary relational information storage step by refining the binary relational information based on the conditions set by the condition setting step.

This shows one example of the binary relational search more specifically. According to this method, the user is allowed to set conditions for all of or one of the targets and relation to be searched, and the stored binary relational information is searched while refining the binary relational information based on the set conditions. Therefore, it is possible to efficiently search for information on a desired target.

The knowledge search method according to still another aspect of the present invention, further includes: a synonym storage step that stores one of the targets and the relation searched and a corresponding synonym while making the synonym correspond to one of the targets and the relation, wherein the search step further includes: a synonym search step that searches the synonym corresponding to one of the targets and the relation searched.

This shows one example of the binary relational search more specifically. According to this method, one of the targets and the relation and the corresponding synonym are stored while making them correspond to each other, and the synonym corresponding to one of the searched targets and relation is searched. Therefore, it is possible to expand the search range up to synonyms and improve knowledge search accuracy.

The knowledge search method according to still another aspect of the present invention, further includes: an ontology storage step that stores one of the targets and the relation searched and a corresponding hierarchical concept item the hierarchical concept item correspond to one of the targets and the relation, wherein the search step further includes: an ontology search step that searches for all of or one of the targets and the relation included in the hierarchical concept item corresponding to one of the targets and the relation searched.

This shows one example of the binary relational search more specifically. According to this method, one of the targets and the relation and the corresponding hierarchical concept item are stored while making them correspond to each other, and all of or one of the targets and the relation included in the hierarchical concept item corresponding to one of the searched targets and relation is searched. Therefore, it is possible to expand the search range up to a generic concept or a subordinate concept and further improve knowledge search accuracy.

The knowledge search method according to still another aspect of the present invention, further includes: a step-execution step that step-executes a binary relation search by the search step based on at leas one of the number of searches done by the search step, the number of targets searches, and hierarchies of the searches.

This shows one example of the binary relational search more specifically. According to this method, the binary relational information is step-executed based on at least one of the number of searches, the number of searched targets, and hierarchies of searches. Therefore, it is possible to execute the processings while the user check the processing results to some extent and selectively conduct a search in a necessary range.

The computer program according to one aspect of the present invention that makes a computer to execute a knowledge search method includes: a binary relational information storage step that stores binary relational information while making a relational source target, a relational destination target, and a relation between the relational source target and the relational destination target correspond to one another; a search condition setting step that allows a user to set a search start target and a search end target; and a search step that searches for all of or one of the targets and the relation for the search start target and the search end target from the binary relational information stored in the binary relational information storage step; wherein knowledge between the search start condition and the search end condition is automatically searched.

According to this computer program, the binary relational information is stored while making the relational source target, the relational destination target, and the relation between the relational source target and the relational destination target correspond to one another, the user is allowed to set the search start target and the search end target, and all of or one of the targets and the relation between the search start target and the search end target are searched from the stored binary relational information stored. Therefore, it is possible to automatically search for the knowledge the search start condition and the search end condition. The search for the information from literatures and the like that is conventionally made manually can be thereby executed by the computer. Therefore, it is possible to promptly, easily, and completely search for information on the information stored in a plurality of large-sized databases.

The computer program according to another aspect of the present invention further includes: a binary relational information creation step that creates the binary relational information based on information on the targets and the relation expressed by a monadic relation or a polynomial relation of three terms or more.

According to this computer program, the binary relational information is created based on the information on the targets and the relation each expressed by a monadic relation or complex polynomial relation of three terms or more. Therefore, it is possible to considerably improve search efficiency by replacing each monadic relation or complex polynomial relation by the binary relation.

The computer program according to still another aspect of the present invention, wherein the search step further includes: a forward search step that searches for the binary relational information stored in the binary relational information storage step based on the relational source target.

This shows one example of the binary relational search more specifically. According to this computer program, the stored binary relational information is searched based on the relational source target. Therefore, it is possible to conduct a search while paying attention to the directivity the binary relation, and a search tracing the target direction in the forward direction.

The computer program according to still another aspect of the present invention, wherein the search step further includes: a backward search step that searches for the binary relational information stored in the binary relational information storage step based on the relational destination target.

This shows one example of the binary relational search more specifically. According to this computer program, the stored binary relational information is searched based on the relational destination target. Therefore, it is possible to conduct a search while paying attention to the directivity of the binary relation, and a search tracing the direction of the target in the backward direction.

The computer program according to still another aspect of the present invention, wherein the search step further includes: a target queue storage step that sequentially stores the search targets in a queue; a breadth-first search step that searches for the binary relational information stored in the binary relational information storage step in an order of storing the searched targets in the queue.

This shows one example of the binary relational search more specifically. According to this computer program, the searched targets are sequentially stored in the queue, and the stored binary relational information is searched in the order of storing the searched target in the queue. Therefore, it is possible to efficiently search for the targets in a wide range.

The computer program according to still another aspect of the present invention wherein the search step further includes: a target overlap elimination step that excludes overlap of the targets stored in the queue, wherein the breadth-first search step conducts a search for the queue from which the overlap of the targets is eliminated by the target overlap elimination step.

This shows one example of the breadth-first search more specifically. According to this computer program, the overlap of the targets stored in the queue is eliminated, and the breadth-first search step searches for the queue from which the overlap of the targets is eliminated. Therefore, it is possible to avoid the infinite loop caused by conducting a search for the same target again.

The computer program according to still another aspect of the present invention, further includes: a condition setting step that allows the user to set the conditions for all of or one of the targets and the relation to be searched, wherein the search step further includes: a condition search step that searches the binary relational information stored in the binary relational information storage step by refining the binary relational information based on the conditions set by the condition setting step.

This shows one example of the binary relational search more specifically. According to this computer program, the user is allowed to set conditions for all of or one of the targets and relation to be searched, and the stored binary relational information is searched while refining the binary relational information based on the set conditions. Therefore, it is possible to efficiently search for information on a desired target.

The computer program according to still another aspect of the present invention, further includes: a synonym storage step that stores one of the targets and the relation searched and a corresponding synonym while making the synonym correspond to one of the targets and the relation, wherein the search step further includes: a synonym search step that searches the synonym corresponding to one of the targets and the relation searched.

This shows one example of the binary relational search more specifically. According to this computer program, one of the targets and the relation and the corresponding synonym are stored while making them correspond to each other, and the synonym corresponding to one of the searched targets and relation is searched. Therefore, it is possible to expand the search range up to synonyms and improve knowledge search accuracy.

The computer program according to still another aspect of the present invention, further includes: an ontology storage step that stores one of the targets and the relation searched and a corresponding hierarchical concept item the hierarchical concept item correspond to one of the targets and the relation, wherein the search step further includes: an ontology search step that searches for all of or one of the targets and the relation included in the hierarchical concept item corresponding to one of the targets and the relation searched.

This shows one example of the binary relational search more specifically. According to this computer program, one of the targets and the relation and the corresponding hierarchical concept item are stored while making them correspond to each other, and all of or one of the targets and the relation included in the hierarchical concept item corresponding to one of the searched targets and relation is searched. Therefore, it is possible to expand the search range up to a generic concept or a subordinate concept and further improve knowledge search accuracy.

The computer program according to still another aspect of the present invention, further includes: a step-execution step that step-executes a binary relation search by the search step based on at leas one of the number of searches done by the search step, the number of targets searches, and hierarchies of the searches.

This shows one example of the binary relational search more specifically. According to this computer program, the binary relational information is step-executed based on at least one of the number of searches, the number of searched targets, and hierarchies of searches. Therefore, it is possible to execute the processings while the user check the processing results to some extent and selectively conduct a search in a necessary range.

The computer readable recording medium according to still another aspect of the present invention that storing the computer program that makes a computer to execute a knowledge search method.

According to this recording medium, the above-mentioned program can be realized with utilizing the computer, by making a computer read and executed a program which is recorded in the recording medium, and same effects of each method described above can be obtained.

### BRIEF DESCRIPTION OF THE DRAWINGS

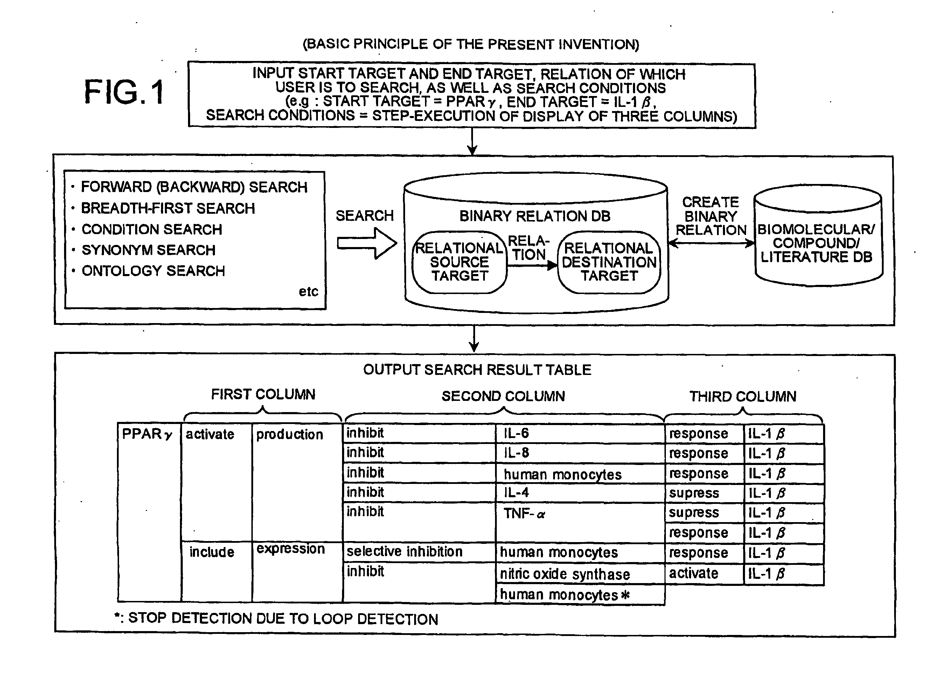

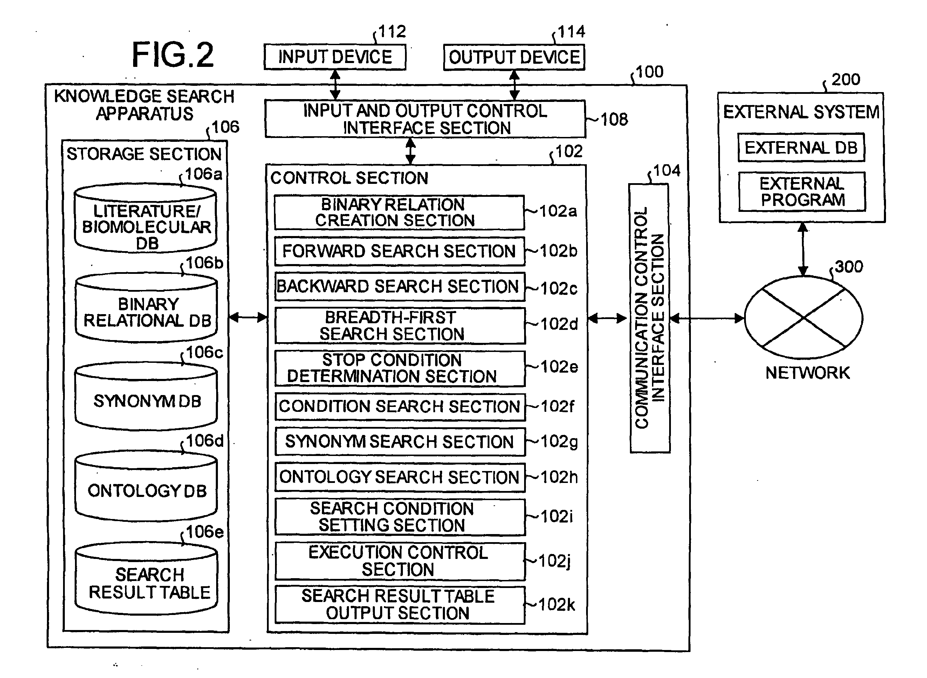

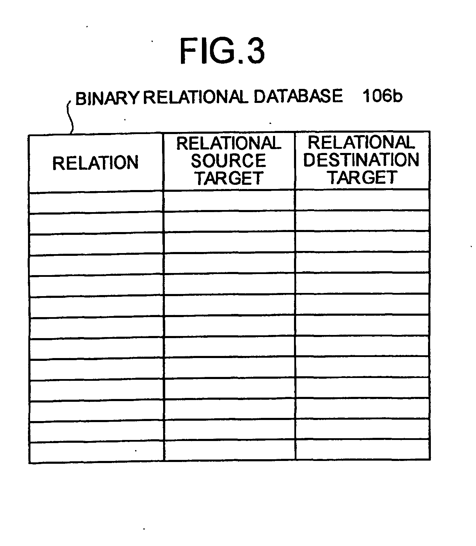

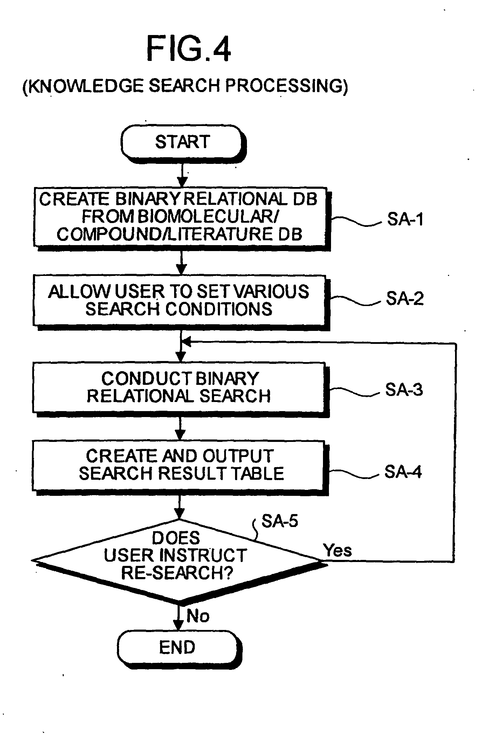

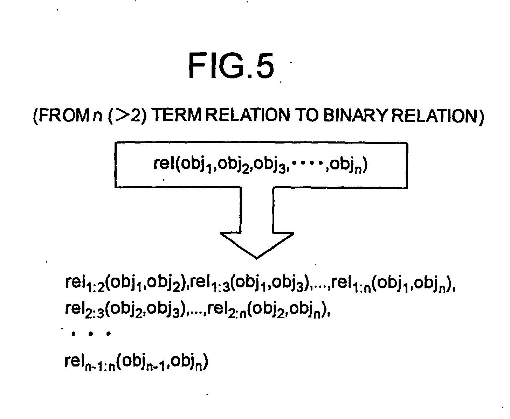

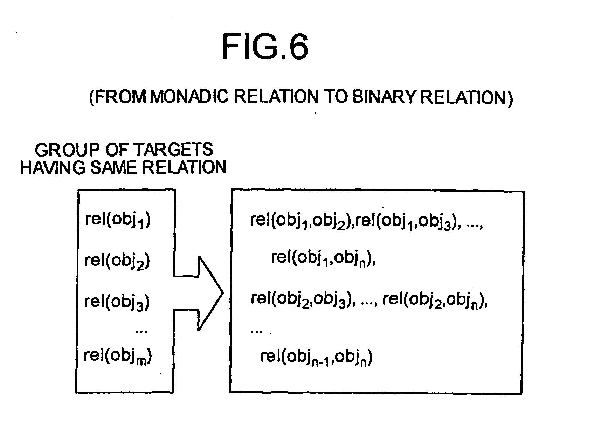

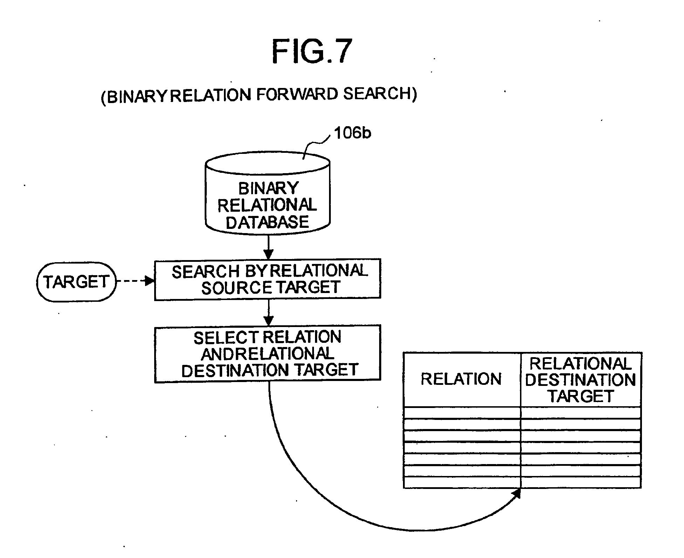

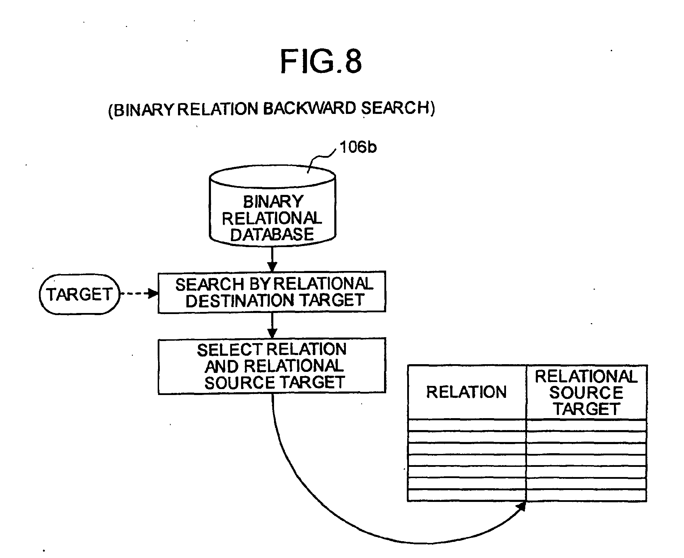

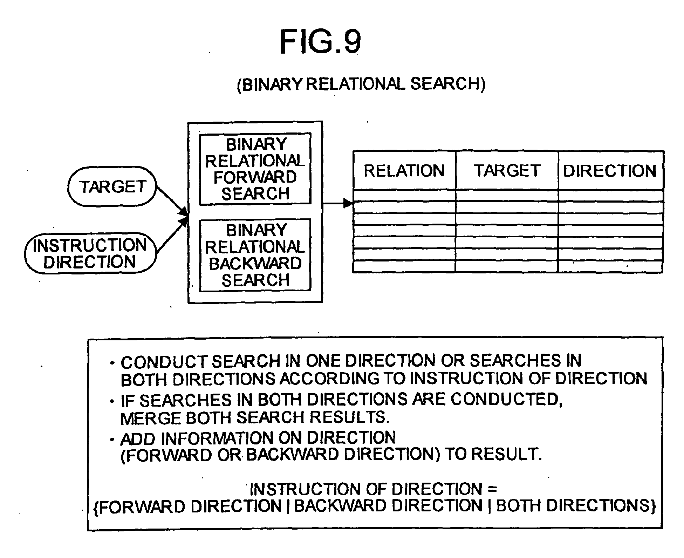

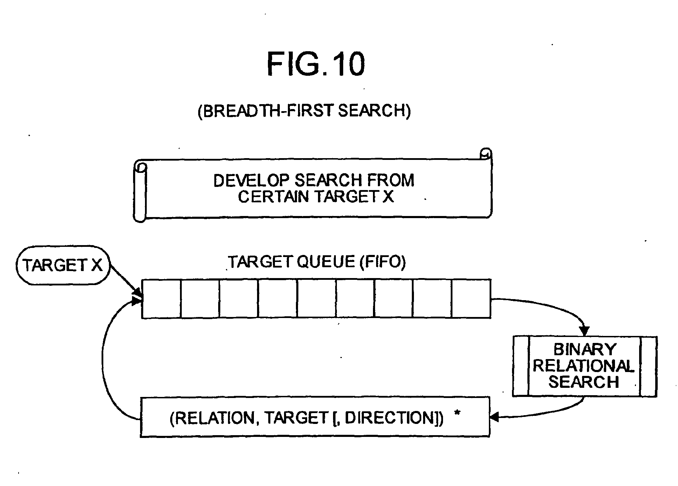

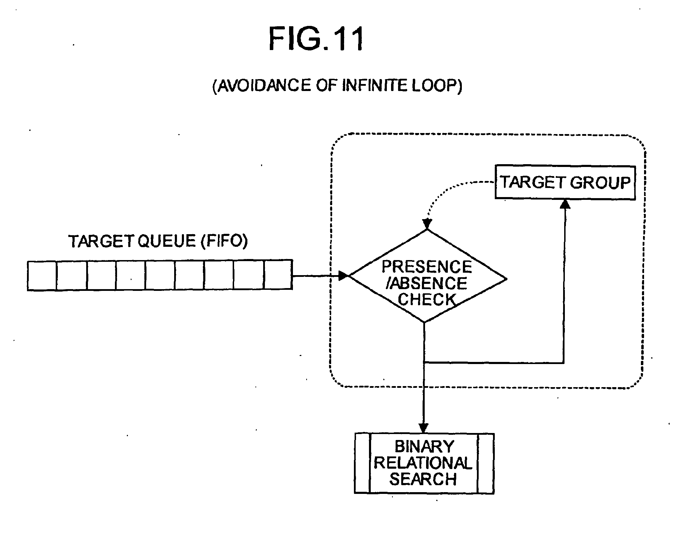

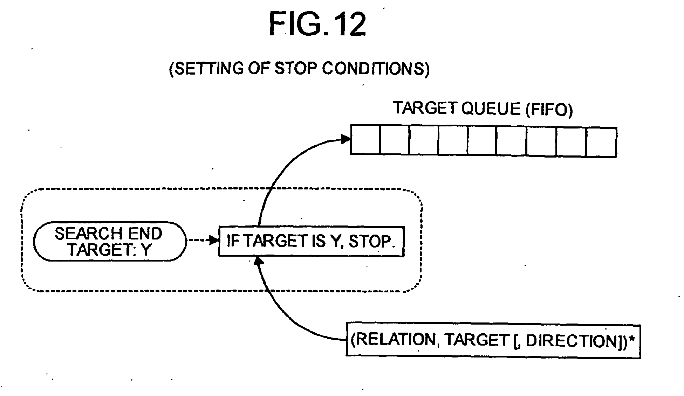

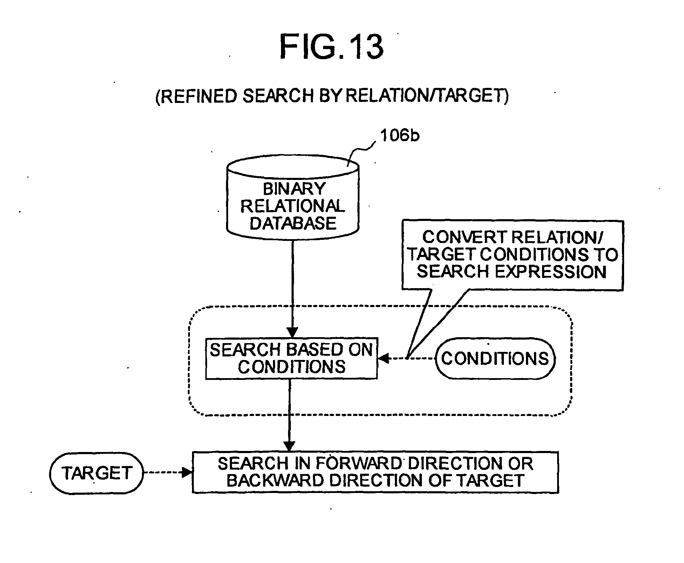

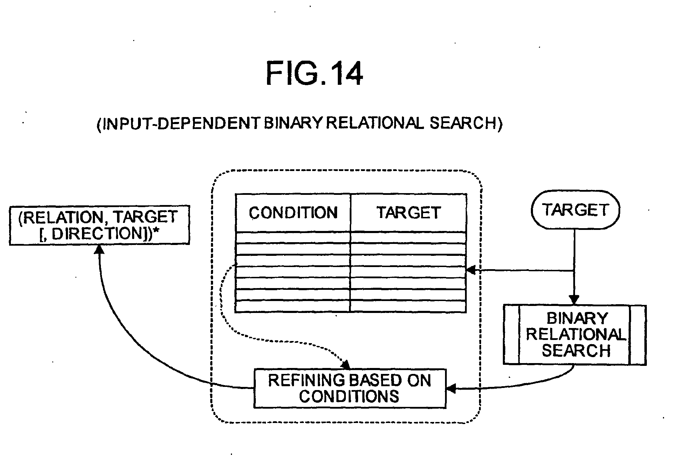

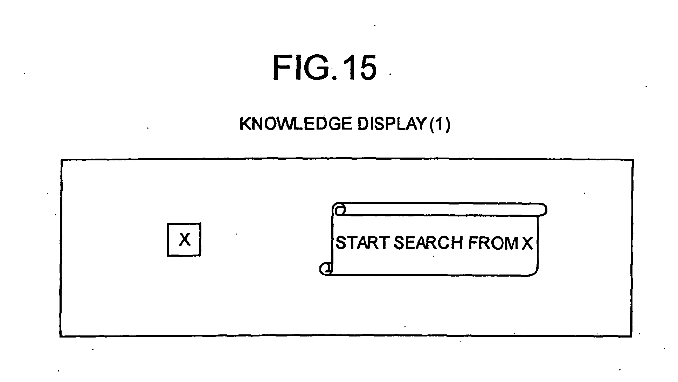

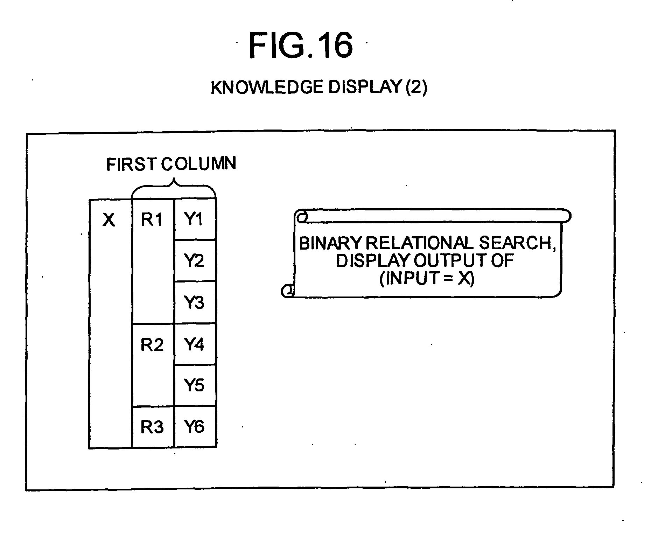

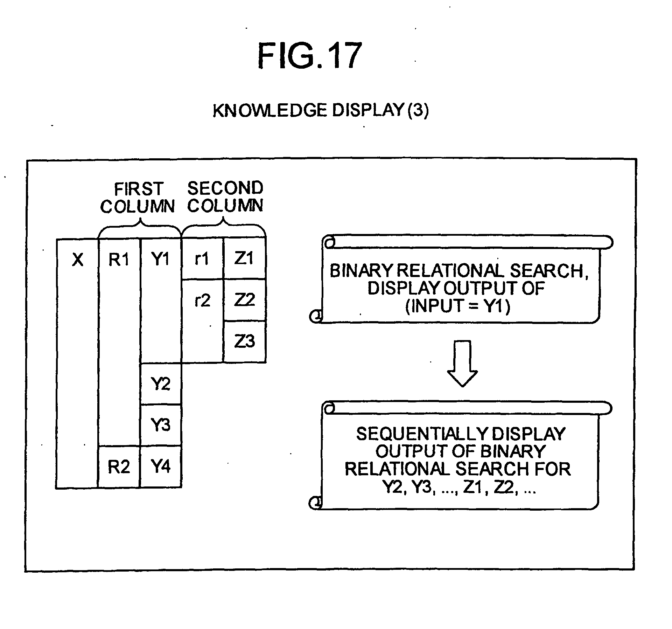

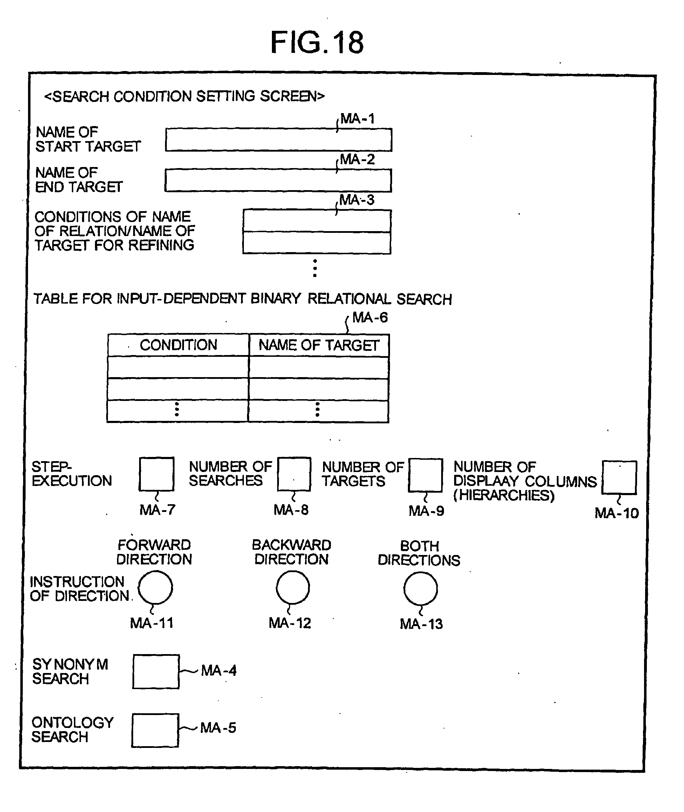

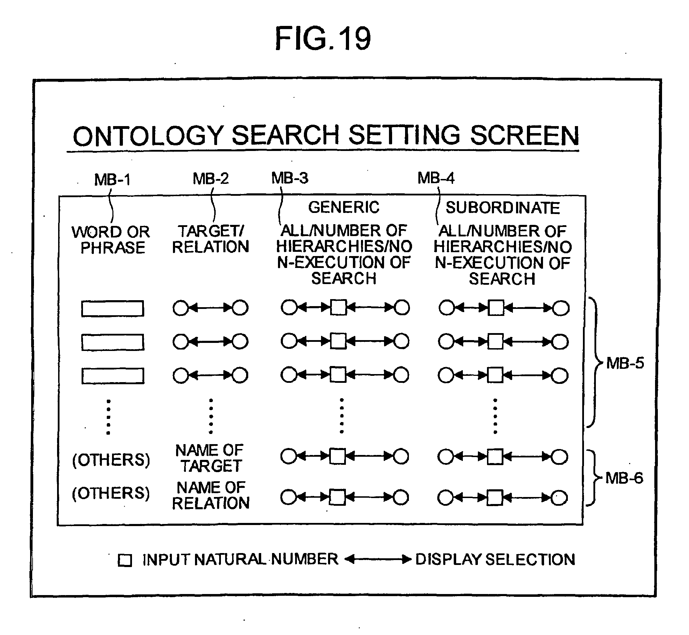

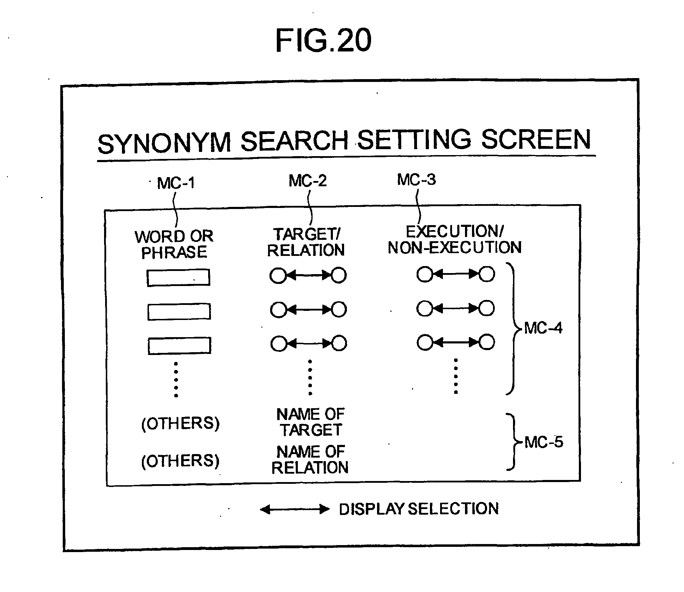

Fig. 1 is a principle block diagram which illustrates the basic principle of the present invention; Fig. 2 is a block diagram which illustrates one example of the configuration of a system to which the present invention is applied; Fig. 3 illustrates one example of information stored in a binary relation database 106b; Fig. 4 is a flow chart which illustrates one example of a knowledge search processing performed by the system according to one embodiment; Fig. 5 is a conceptual view which illustrates one example in which the binary relation creation section 102a creates binary relational information based on information on a target and a relation expressed by a polynomial relation of three terms or more; Fig. 6 is a conceptual view which illustrates one example in which a binary relation creation section 102a creates binary relational information based on information on targets and a relation expressed by monadic relations; Fig. 7 is a conceptual view which illustrates one example of a forward search processing performed by a forward search section 102b; Fig. 8 is a conceptual view which illustrates one example of a backward search processing performed by a backward search section 102c, Fig. 9 is a conceptual view which illustrates one example of a search processing performed by a knowledge search apparatus 100 if the search processing is performed by a combination of a forward search and a backward search; Fig. 10 is a conceptual view which illustrates one example of a breadth-first search processing performed by a breadth-first search section 102d; Fig. 11 is a conceptual view which illustrates one example of a infinite loop avoidance processing performed by the breadth-first search section 102d; Fig. 12 is a conceptual view which illustrates one example of a stop condition determination processing performed by a stop condition determination section 102e; Fig. 13 is a conceptual view which illustrates one example of a refined search processing performed by a condition search section 102f based on the relation or the target; Fig. 14 is a conceptual view which illustrates one example of an input-dependent binary relational search processing performed by the condition search section 102f; Fig. 15 is a conceptual view which illustrates one example of a display screen of a search result table displayed on an output device 114 of the knowledge search apparatus 100; Fig. 16 is a conceptual view which illustrates one example of a display screen of the search result table displayed on the output device 114 of the knowledge search apparatus 100; Fig. 17 is a conceptual view which illustrates one example of the display screen of the search result table displayed on the output device 114 of the knowledge search apparatus 100; Fig. 18 is a conceptual view which illustrates one example of a search condition setting screen displayed on the output device 114 of the knowledge search apparatus 100; Fig. 19 is a conceptual view which illustrates one example of an ontology search condition setting screen displayed on the output device 114 of the knowledge search apparatus 100; and Fig. 20 is a conceptual view which illustrates one example of a synonym search condition setting screen displayed on the output device 114 of the knowledge search apparatus 100.

### BEST MODE FOR CARRYING OUT THE INVENTION

Exemplary embodiments of a knowledge search apparatus, a knowledge search method, a program, and a recording medium according to the present invention will be explained hereinafter in detail with reference to the accompanying drawings. It should be noted that this invention is not limited to the embodiments.

In the embodiments, an instance of applying the present invention to a search system in the biotechnology field will be explained. However, the present invention is not limited to this search system but may be similarly applied to all other systems for searching for information accumulated in databases.

### [Outline of the Invention]

The outline of the present invention will be explained first, followed by the configuration, processings, and the like of the present invention. Fig. 1 is a principle block diagram which illustrates the basic principle of the present invention.

Roughly, the present invention has the following basic features. A user designates first two targets a relation between which the user is to search for as a search start target and a search end target, respectively. The user also designates various search conditions. In Fig. 1, an instance of searching for knowledge between PPARγ and interleukin 1β (IL-1β) is illustrated. Fig. 1 also illustrates the search condition under which up to three search hierarchies are step-executed.

According to the present invention, a binary relation database that stores binary relational information extracted from various type of biomolecular databases (e.g., databases that store sequence information on DNAs and the like, and structural information on proteins and the like), literature databases (e.g., databases that store technical literatures and papers) is accessed, and the knowledge between the search start target and the search end target is automatically searched.

The "binary relational information" includes a relational source target, a relational destination target, and a relation between the targets. The "target" means herein a name or the like of a matter, a status, or the like, and examples of the "target" include names of biomolecules such as proteins, names of internal organs, and names of diseases. The "relation" indicates the relation between the targets, and examples of the "relation" include interaction, expression, suppression, promotion, cause, and property. According to the present invention, binary relational information is created from pieces of knowledge (information) stored in the biomolecular databases, the compound databases, the literature databases, and the like, using a known structural analysis technique, a natural language processing technique, or the like, and stored in the binary relation database. The binary relation database can be searched by using any one of the relation, the relational source target, and the relational destination target as a key. In addition, any one of or all of the relation, the relational source target, and the relational destination target can be selected.

The search method according to the present invention may be conducted using any one of the following methods or by appropriately combining the following methods.

### (1) Forward Search

Based on the relational source target, the binary relational information stored in the binary relation database is searched. Namely, a search is conducted based on the relational source target, and the relational destination target and the relation are acquired.

### (2) Backward Search

Based on the relational destination target, the binary relational information stored in the binary relation database is searched. Namely, a search is conducted based on the relational destination target, and the relational source target and the relation are acquired.

### (3) Breadth-First Search

Searched targets are sequentially stored in a queue, and the binary relational information stored in the binary relation database is searched in an order of storing the targets in the queue. In addition, in order to avoid infinite loop, overlap of the targets stored in the queue is eliminated, and the queue from which the overlap is eliminated is searched.

### (4) Condition search

The user is allowed to set conditions for all of or one of the search targets and the relation, and a search is conducted while refining the binary relational information stored in the binary relation database based on the set conditions.

### (5) Synonym Search

A search is conducted while expanding a search range up to synonyms corresponding to one of the targets or the relation.

### (6) Ontology Search

A search is conducted while expanding the search range up to other targets or relations included in a hierarchical concept corresponding to one of the targets or the relation.

According to the present invention, search results are summed up in a search result table in the form of a table and output. In the search result table shown in Fig. 1, the search start target is displayed in a left column, and search results of the search start target (relations and relational destination targets) are hierarchically displayed.

The uppermost search result shown in Fig. 1, for example, will be explained in detail. According to the present invention, with the search start target "PPARγ" used as a key, the relational source target in the binary relation database is searched first, and the corresponding relation (activate) and the relational destination target (production) are acquired. According to the present invention, with "production" used as a key, the relational source target in the binary relation database is searched next, and the corresponding relation (inhibit) and the relational destination target (IL-1β: search end target) are acquired.

Furthermore, according to the present invention, a row that indicates a search which cannot arrive at the search end target or IL-1β may not be output. Further, in order to avoid the infinite loop, a part which is not be searched may be given a mark ("*" in Fig. 1). In addition, link information on an extraction source database and the like may be set to the respective relations and targets in the table. By doing so, if the user selects a desired relation or target in the table, a content (e.g., the original of a literature or annotation information) of the extraction source database may be displayed on the display screen.

Additionally, by executing a relational search with TNF-α discovered as explained above set as a new start target and PPARγ set as the end target, a chain between the relation (activate) and the relational destination target (p38), that between the relation (phosphorylate) and the relational destination target (PGC-1), or that between the relation (activate) and the relational destination target (PPARγ), for example, can be discovered.

### [System Configuration]

The configuration of the present system will first be explained. Fig. 2 is a block diagram which illustrates one example of the configuration of the system to which the present invention is applied. In Fig. 2, among constituent elements of the system, only those related to the present invention are conceptually illustrated. The system is constituted so that a knowledge search apparatus 100 is communicably connected to an external system 200 that provides external databases about literatures, biomolecules, and the like, external programs for searches, and the like through a network 300.

In Fig. 2, the network 300 functions to connect the knowledge search apparatus 100 and the external system 200 to each other, and is, for example, the Internet.

In Fig. 2, the external system 200 is connected to the knowledge search apparatus 100 through the network 300, and functions to provide the user with the external databases about the literatures and biomolecules, and websites for executing external programs for searches and the like.

The external system 200 may be constituted as a WEB server, an ASP server, or the like, and hardware of the external system 200 may be constituted by an information processing apparatus such as a commercially available workstation or personal computer, and accessories of the apparatus. Respective functions of the external system 200 are realized by a CPU, a disk device, a memory device, an input device, an output device, a communication control device, and the like in the hardware configuration of the external system 200 as well as programs for controlling these devices, and the like.

In Fig. 2, the knowledge search apparatus 100 roughly includes a control section 102 such as the CPU for generally controlling entirety of the knowledge search apparatus 100, a communication control interface section 104 connected to a communication apparatus (not illustrated) such as a router connected to a communication line or the like, an input and output control interface 108 connected to the input device 112 and the output device 114, and a storage section 106 that stores various databases and tables. The respective sections are communicably connected to one another through arbitrary communication lines. In addition, this knowledge search apparatus 100 is communicably connected to the network 300 through the communication device such as the router and a wired or wireless communication line such as a dedicated line.

The various databases and tables (a biomolecular/compound/literature database 106a to a search result table 106e) are storage units for a fixed disk device or the like, and store various programs, tables, files, databases, webpage files, and the like used for the various processings.

Among the constituent elements of the storage section 106, the biomolecular/compound/literature database 106a is a database that stores information on literatures, biomolecules, and the like.

A binary relation database 106b is a binary relational information storage unit that stores binary relational information for storing the relational source target; the relational destination target, and the relation between the targets while making them correspond to one another. Fig. 3 illustrates one example of the information stored in the binary relation database 106b.

As illustrated in Fig. 3, the information stored in this binary relation database 106b includes the relational source target, the relational destination target, and the relation between the targets, while making them correspond to one another.

A synonym database 106c is a synonym storage unit that stores one of the targets or the relation, and a corresponding synonym while making them correspond to each other.

An ontology database 106d is an ontology storage unit that stores one of the targets or the relation, and corresponding hierarchical concept items while making them correspond to each other. The hierarchical concept items are of a tree data structure with one of the relation and targets set as a root. A generic concept item and a subordinate concept item are managed while making them correspond to each other. For example, as for the hierarchical concept items for the target "interieukin", the generic concept item includes "cytokine" or the like and the subordinate concept item includes "interleukin-1" or the like. These items are made to correspond to each other in the tree structure.

The biomolecular/compound/literature database 106a to the ontology database 106d may be external databases accessed through the Internet, or in-house databases created by copying the databases, storing original information, or adding individual annotation information and the like to the databases.

The search result table 106e is a table that displays search results obtained by the knowledge search apparatus 100 in the form of a table.

In Fig. 2, the communication control interface section 104 controls the communication between the knowledge search apparatus 100 and the network 300 (or the communication device such as the router). Namely, the communication control interface 104 functions to communicate data with other terminals through communication lines.

In Fig. 2, the input and output control interface section 108 controls the input device and the output device. As the output device 114, a monitor (including a home television set), a loudspeaker or the like can be used (it is noted that the output device 114 is sometimes referred to as "monitor" hereafter). As the input device 112, a keyboard, a mouse, a microphone, or the like can be used. The monitor realizes a pointing device function in cooperation with the mouse.

In Fig. 2, the control section 102 includes an internal memory for storing various programs such as an OS (Operating System), programs for specifying various processing procedures, and required data. Using these programs and the like, information processings for executing various processings are performed. The control section 102 functionally conceptually includes the binary relation creation section 102a, the forward search section 102b, the backward search section 102c, the breadth-first search section 102d, the stop condition determination section 102e, the condition search section 102f, a synonym search section 102g, an ontology search section 102h, a search condition setting section 102i, an execution control section 102j, and a search result table output section 102k.

Among these sections, the binary relation creation section 102a is a unit that creates the binary relational information from the biomolecular/compound/literature database 106a, and that stores the created binary relational information in the binary relation database 106b, and is a binary relational information creation unit that creates the binary relational information based on information on the targets and the relation each expressed by a monadic relation or a polynomial relation of three terms or more. The forward search section 102b is a forward search unit that searches for the binary relational information stored in the binary relational storage unit based on the relational source target.

The backward search section 102c is a backward search unit that searches for the binary relational information stored in the binary relational information storage unit based on the relational destination target. The breadth-first search section 102d is a target queue storage unit that sequentially stores searched targets in the queue, a breadth-first search unit that searches for the binary relational information stored in the binary relational information storage unit in the order of storing the targets in the queue, and is a target overlap elimination unit that eliminates overlap of the targets stored in the queue.

The stop condition determination section 102e is a stop condition determination unit that determines whether a search result coincides with the search end target or preset stop conditions. The condition search section 102f is a condition search unit that searches for the binary relational information stored in the binary relational information storage unit by refining the binary relational information based on the conditions set by the condition setting unit. The synonym search section 102g is a synonym search unit that searches a synonym corresponding to one of the searched targets and relation. The ontology search section 102h is an ontology search unit that searches for all of or one of the targets and the relation included in the hierarchical concept items corresponding to one of the searched targets or relation.

The search condition setting section 102i is a search condition setting unit that allows the user to set the search start target and the search end target, and a condition setting unit that allows the user to set conditions for all of or one of the searched targets and relations. The execution control section 102j is a step execution unit that step-executes the searches by the search units based on at least one of the number of searches done by the search units, the number of searched targets, and hierarchies of searches. In addition, the search result table output section 102k is a search result table output unit that outputs the search result table for automatically searching for the knowledge between search start conditions and search end conditions. The processings performed by the respective sections will be explained later in detail.

The forward search section 102b, the backward search section 102c, the breadth-first search section 102d, the stop condition determination section 102e, the condition search section 102f, the synonym search section 102g, and the ontology search section 102h constitute a search unit that searches for all of or one of the targets and the relation between the search start target and the search end target from the binary relational information stored in the binary relational information storage unit.

### [Processings of the System]

One example of the processings of the system according to this embodiment constituted as explained above will be explained in detail with reference to Figs. 4 to 18.

### [Knowledge Search Processing]

The detail of the knowledge search processing will first be explained with reference to Figs. 4 to 14 and Fig. 18. Fig. 4 is a flow chart which illustrates one example of the knowledge search processing performed by the system in this embodiment.

The knowledge search apparatus 100 first accesses the biomolecular/compound/literature database 106a, acquires information, and creates the binary relational information based on knowledge in the information by the processing performed by the binary relation creation section 102a (at a step SA-1). Fig. 5 is a conceptual view which illustrates one example in which the binary relation creation section 102a creates the binary relational information based on the information on the target and the relation each expressed by a polynomial relation of three terms or more. As illustrated in Fig. 5, the binary relation creation section 102a creates a binary relation by making all permutation combinations for the polynomial relation. Fig. 6 is a conceptual view which illustrates one example in which the binary relation creation section 102a creates the binary relational information based on information on the targets and the relations each expressed by a monadic relation. An example in which the target is expressed by the monadic relation includes expressing a target property by a relation. As illustrated in Fig. 6, the binary relation creation section 102a creates a group in which targets having the same relation are collected for the polynomial relation, and creates the binary relation by making all permutation combinations of the targets included in this group.

The knowledge search apparatus 100 allows the user to set various search conditions by the processing performed by the search condition setting section 102i (at a step SA-2). Fig. 18 illustrates one example of a search condition setting screen displayed on the output device 114 of the knowledge search apparatus 100. As illustrated in Fig. 18, the search condition setting screen is constituted to include, for example, an input area for the search start target MA-1, an input area for the search end target MA-2, an input area for relational or target conditions for refining MA-3, a synonym search selection button for selecting a synonym search MA-4, an ontology search selection button for selecting an ontology search MA-5, a condition setting table for an input-dependent binary relational search MA-6, a selection button for selecting step-execution MA-7, input areas for setting stop conditions for the step-execution (MA-8 to MA-10), a selection button for selecting a forward direction as a search direction MA-11, a selection button for selecting a backward direction as the search direction MA-12, and a selection button for selecting both directions as the search directions MA-13.

If the user selects the synonym search selection button MA-4 through the input device 112, the search condition setting section 102i outputs a synonym search condition setting screen illustrated in Fig. 20 to a monitor. If the user selects the ontology search selection button MA-5 through the input device 112, the search condition setting section 102i outputs an ontology search condition setting screen illustrated in Fig. 19 to the monitor.

Fig. 19 illustrates one example of the ontology search condition setting screen displayed on the output device 114 of the knowledge search apparatus 100. As illustrated in Fig. 19, the ontology search condition setting screen is constituted to include, for example, an input area for a word or phrase that serves as an ontology search target MB-1, a selection area for selecting whether the word or phase is a target or a relation MB-2, an input area for search conditions for higher ontology (e.g., designation of all generic concepts, designation of up to desired higher hierarchies, or non-designation of generic concepts) MB-3, an input area for search conditions for lower ontology (e.g., designation of all subordinate concepts, designation of up to desired lower hierarchies, or non-designation of subordinate concepts) MB-4, an input area for each arbitrary word or phase MB-5, an input area for default targets and relation MB-6, and the like.

Fig. 20 illustrates one example of the synonym condition setting screen displayed on the output device 114 of the knowledge search apparatus 100. As illustrated in Fig. 20, the synonym search condition setting screen is constituted to include, for example, an input area for a word or phase as a synonym search target MC-1, a selection area for selecting whether the word or phase is a target or a relation MC-2, a selection area for determining whether a synonym search is done MC-3, an input area for each arbitrary word or phase MC-4, and an input area for default targets and relation MC-5.

If the user sets desired conditions through the input device 112 while checking each screen output to the output device 114, the search condition setting section 102i stores a content of the setting in a predetermined storage area of the storage section 106.

The knowledge search apparatus 100 searches for all of or one of the targets and the relation between the search start target and the search end target from the binary relational information stored in the binary relation database 106b (at a step SA-3). Namely, the knowledge search apparatus 100 accesses the binary relation database 106b, and automatically searches for the knowledge between the search start target and the search end target from the binary relation database 106b.

The search method according to the present invention may be conducted using any one of the following methods or by appropriately combining the following methods.

### (1) Forward Search

Based on the relational source target, the binary relational search section 102b searches for the binary relational information stored in the binary relation database 106b. Fig. 7 is a conceptual view which illustrates one example of the forward search processing performed by the forward search section 102b. As illustrated in Fig. 7, the forward search section 102b searches the binary relation database 106b based on the relational source target, and acquires the relational destination target and the relation.

### (2) Backward Search

Based on the relational destination target, the backward search section 102c searches for the binary relational information stored in the binary relation database 106b. Fig. 8 is a conceptual view which illustrates one example of the backward search processing performed by the backward search section 102c. As illustrated in Fig. 8, the backward search section 102c searches the binary relation database 106b based on the relational destination target, and acquires the relational source target and the relation.

Fig. 9 is a conceptual view which illustrates one example of the search processing performed by the knowledge search apparatus 100 if the search processing is performed by a combination of a forward search and a backward search. As illustrated in Fig. 9, the knowledge search apparatus 100 can conduct either a search in one direction (forward or backward direction) or searches in both directions in accordance with a user's instruction of direction. If the knowledge search apparatus 100 conducts searches in the both directions, both search results may be merged and information indicating each search direction may be added as a search result.

### (3) Breadth-First Search

The breadth-first search section 102d sequentially stores searched targets in a queue, and searches for the binary relational information stored in the binary relation database 106b in the order of storing the targets in the queue. Fig. 10 is a conceptual view which illustrates one example of the breadth-first search processing performed by the breadth-first search section 102d. As illustrated in Fig. 10, the breadth-first search section 102d sequentially stores searched targets (targets X) in a first-in first-out (hereinafter, "FIFO") queue, searches for the binary relational information stored in the binary relation database 106b in the order of storing the targets in the queue, and acquires relations and targets. The breadth-first search section 102d also sequentially stores the newly acquired targets in the queue.

Fig. 11 is a conceptual view which illustrates one example of the infinite loop avoidance processing performed by the breadth-first search section 102d. As illustrated in Fig. 11, the breadth-first search section 102d creates a group of, for example, the targets stored in the queue and checks whether each target extracted from the queue is present in the group so as to avoid infinite loop. The breadth-first search section 102d can thereby eliminate overlap of the targets stored in the queue and conduct a search for the queue from which the overlap is eliminated.

### (4) Stop Condition Determination

The stop condition determination section 102e determines whether the search result coincides with the search end target or preset stop conditions. Fig. 12 is a conceptual view which illustrates one example of the stop condition determination processing performed by the stop condition determination section 102e. As illustrated in Fig. 12, the stop condition determination section 102e determines, for example, whether the target extracted from the target queue or the acquired target or relation coincides with the search end target. If they coincide, the stop condition determination section 102e stops the search processing.

### (5) Condition Search

The condition search section 102f conducts a search while refining the binary relational information stored in the binary relation database 106b based on conditions for all of or one of the search targets and the relation set by the user using the search conditions setting section 102i. Fig. 13 is a conceptual view which illustrates one example of the refined search processing performed by the condition search section 102f by the relation or the targets. As illustrated in Fig. 13, the condition search section 102f refines the search range based on the conditions set by the user in advance for the binary relation database 106b, and conducts the search within the range. For example, if the search start target is set at the "protein A", the search end target is set at the "protein B", and the condition is set at "human beings", then the condition search section 102f searches for the knowledge from the protein A to the protein B within the range of the targets and the relation about the "human beings".

Fig. 14 is a conceptual view which illustrates one example of the input-dependent binary relational search processing performed by the condition search section 102f. As illustrated in Fig. 14, the condition search section 102f creates a search expression using the conditions, the relation, the targets, and the direction based on the table (MA-6 in Fig. 18) for the input-dependent binary relational search set by the user in advance, and conducts a search.

### (6) Synonym Search

The synonym search section 102g conducts a search while expanding the search range up to the synonyms corresponding to one of the targets or the relation. The knowledge search apparatus 100 prepares the synonym database 106c in advance, and expands the search range up to the synonyms using the synonym database 106c when one of the targets or the relation is used. The designation of the expansion of the search range up to the synonyms may be made by the user for each used part or made for each relation or target.

### (7) Ontology Search

The ontology search section 102h conducts a search while expanding the search range up to other targets or relations included in the hierarchical concepts corresponding to one of the targets or the relation. Namely, the knowledge search apparatus 100 prepares the ontology database 106d in advance, and expands the search range up to higher or lower ontology (conceptual items) using the ontology database 106d when the relation or the target is used. The designation of the expansion of the search range up to the ontology may be made by the user for each used part or made for each relation or target. In addition, the designation may be made not only such that all generic concept words or phrases or all subordinate concept words or phrases of a certain target or relation are designated but also such that hierarchies are designated up to higher n hierarchies or lower n hierarchies.

The knowledge search apparatus 100 creates the search result table and outputs the created search result table to the output device 114 by the processing performed by the search result table output section 102k (at a step SA-4).

The user checks the content of the search result table. If the user instructs a search again to deeper hierarchies (at a step SA-5), the processing returns to the step SA-3 at which the search processing is re-executed. The knowledge search processing is thereby finished.

### [Step Execution Processing]

The detail of the step-execution processing will be explained with reference to Figs. 15 to 17.

Since the database search is automatically conducted and lots of data processing time is required, the step-execution for executing the processing while the user checks the processing result to some extent is quite effective. The execution control section 102j step-executes the searches by the search units based on at least one of the number of searches done by the search units, the number of searched targets, and the hierarchies of searches. One example of the step-execution processing will be explained.

Fig. 15 illustrates one example of a display screen of the search result table displayed on the output device 114 of the knowledge search apparatus 100. As illustrated in Fig. 15, on the display screen of the search result table, only the first search start target is displayed. If the user designates a search, the search processing for searching the binary relation database 106b is performed and the search result table is updated.

Fig. 16 illustrates one example of the display screen of the search result table displayed on the output device 114 of the knowledge search apparatus 100. As illustrated in Fig. 16, on the display screen of the search result table, the first search start target and the search result (relations and targets) in the first column are displayed. If the user designates a re-search, the search processing for searching the binary relation database 106b is performed and the search result table is updated.

Fig. 17 illustrates one example of the display screen of the search result table displayed on the output device 114 of the knowledge search apparatus 100. As illustrated in Fig. 17, on the display screen of the search result table, the first search start target, the search result (relations and targets) in the first column, and the search result (relations and targets) for Y1 are displayed. If the user designates a re-search, the search processing for searching the binary relation database 106b is performed for Y2 or the like and the search result table is updated.

In addition, the execution control section 102j controls the target queue. If the user designates a specific target for which the search result is to be further displayed on the search result table, then the execution control section 102j moves the selected target to a top of the target queue and conducts a search for the target. If the user designates a target to be deleted from the search result on the search result table, the execution control section 102j deletes the selected target from the target queue. The step-execution processing is thereby finished.

### [Other Embodiments]

Exemplary embodiments of the present invention have been explained so far. However, the present invention may be carried out by not only these embodiments but also various other embodiments within the scope of the technical spirit according to the appended claims.

For example, the example in which the knowledge search apparatus 100 performs processings in a stand-alone mode has been explained. Alternatively, the knowledge search apparatus 100 may perform each processing in response to a request from a client terminal constituted separately from the knowledge search apparatus 100, and may return the processing result to the client terminal.

Further, among the respective processings explained in the embodiment, all of or part of the processings explained to be performed automatically may be performed manually or all of or part of the processings explained to be performed manually may be performed automatically by a well-known method.

The processing procedures, the control procedures, the concrete names, the information including various pieces of registered data and the parameters for search conditions and the like, the screen examples, and the database configurations explained above or illustrated in the drawings may be arbitrarily changed unless specified otherwise.

Furthermore, the respective constituent elements of the knowledge search apparatus 100 illustrated in the drawings are functionally conceptual, and the knowledge search apparatus 100 is not always required to be physically constituted as illustrated in the drawings.

For instance, all of or arbitrary part of the processing functions of the respective sections (respective devices) of the knowledge search apparatus 100, particularly the respective processing functions performed by the control section can be realized by the Central Processing Unit (CPU) and programs interpreted and executed by the CPU, or can be realized as hardware based on wired logic. The programs are recorded on the recording medium to be explained later, and mechanically read by the knowledge search apparatus as 100 needed.

Namely, a computer program for issuing commands to the CPU in cooperation with an OS and for performing various processings is recorded on the storage section 106 such as a ROM or an HD. This computer program is executed by being loaded by a RAM, and the computer program as well as the CPU constitutes the control section. Alternatively, this computer program may be recorded on an application program server connected to the knowledge search apparatus 100 through an arbitrary network, and the computer program can be downloaded either entirely or partially as needed.

Further, the program according to the present invention can be stored in a computer readable recording medium. It is assumed herein that examples of this "recording medium" include arbitrary "portable physical mediums" such as a flexible disk, a magneto-optical disk, a ROM, an EPROM, an EEPROM, a CD-ROM, an MO, and a DVD, arbitrary "fixed physical mediums" such as a ROM, a RAM, and an HD included in various computer systems, and "communication mediums" that temporarily hold the program such as a communication line or a carrier wave used when the program is transmitted through the network represented by a LAN, a WAN, or the Internet.

The "program" is a data processing method described in an arbitrary language or by an arbitrary description method, and the form of the "program" is not limited but may be a source code, a binary code, or the like. The "program" is not limited to a program constituted as a single program. Examples of the "program" include a program constituted to be distributed as a plurality of modules or libraries, and a program that fulfils its function in cooperation with another program represented by the OS The concrete configurations, reading procedures, install procedures after reading, and the like of the respective devices illustrated in the embodiment for reading the recording medium may be well-known configurations and procedures.

The various databases and the like (the biomolecular/compound/literature database 106a to the search result table 106e) stored in the storage section 106 are storage units for a memory device such as a RAM or a ROM, a fixed disk device such as a hard disk, a flexible disk, an optical disk, and the like. The various databases and the like store various programs, tables, files, databases, webpage files, and the like used for various processings and provision of websites.

The knowledge search apparatus 100 may be realized by connecting peripherals such as a printer, a monitor, and an image scanner to an information processing apparatus such as an information processing terminal, e.g., a well-known personal computer or workstation, and by installing software (including a program, data, or the like) for realizing the method of the present invention into the information processing apparatus.

The concrete form of distribution and integration of the knowledge search apparatus 100 is not limited to that illustrated in the drawings. All of or part of the knowledge search apparatus 100 can be functionally or physically distributed or integrated in arbitrary units according to various loads and the like. For example, each database may be constituted independently as an independent database device, and part of the processings may be realized using a CGI (Common Gateway Interface).

Furthermore, the network 300 functions to connect the knowledge search apparatus 100 and the external system 200 to each other, and may include any one of, for example, the Internet, the Intranet, a LAN (which may be either wired or wireless), a VAN, a personal computer communication network, a public telephone network (which may be either analog or digital), a dedicated line network (which may be either analog or digital), a CATV network, a portable line exchange network/portable packet exchange network such as an IMT 2000 network, a GSM network, or a PDC/PDC-P network, a wireless call network, a local wireless network such as Bluetooth, a PHS network, and satellite communications network such as CS, BS, or ISDB. That is, the present system can transmit and receive various pieces of data through an arbitrary network whether the system is wired or wireless.

As explained so far in detail, according to the present invention, the binary relational information is stored while making the relational source target, the relational destination target, and the relation between the relational source target and the relational destination target correspond to one another, the user is allowed to set the search start target and the search end target, and all of or one of the targets and the relation between the search start target and the search end target are searched from the stored binary relational information stored. Therefore, it is possible to provide the knowledge search apparatus, the knowledge search method, the program, and the recording medium capable of automatically searching for the knowledge the search start condition and the search end condition.

According to the present invention, the search for the information from literatures and the like that is conventionally made manually can be executed by the computer. Therefore, it is possible to provide the knowledge search apparatus, the knowledge search method, the program, and the recording medium capable of promptly, easily, and completely searching for information on the information stored in a plurality of large-sized databases.

According to the present invention, the binary relational information is created based on the information on the targets and the relation each expressed by a monadic relation or complex polynomial relation of three terms or more. Therefore, it is possible to provide the knowledge search apparatus, the knowledge search method, the program, and the recording medium capable of considerably improving search efficiency by replacing each monadic relation or complex polynomial relation by the binary relation.

According to the present invention, the stored binary relational information is searched based on the relational source target. Therefore, it is possible to provide the knowledge search apparatus, the knowledge search method, the program, and the recording medium capable of conducting a search while paying attention to the directivity the binary relation, and a search tracing the target direction in the forward direction.

According to the present invention, the stored binary relational information is searched based on the relational destination target. Therefore, it is possible to provide the knowledge search apparatus, the knowledge search method, the program, and the recording medium capable of conducting a search while paying attention to the directivity of the binary relation, and a search tracing the direction of the target in the backward direction.

According to the present invention, the searched targets are sequentially stored in the queue, and the stored binary relational information is searched in the order of storing the searched target in the queue. Therefore, it is possible to provide the knowledge search apparatus, the knowledge search method, the program, and the recording medium capable of efficiently searching for the targets in a wide range.

According to the present invention, the overlap of the targets stored in the queue is eliminated, and the breadth-first search unit searches for the queue from which the overlap of the targets is eliminated. Therefore, it is possible to provide the knowledge search apparatus, the knowledge search method, the program, and the recording medium capable of avoiding the infinite loop caused by conducting a search for the same target again.

According to the present invention, the user is allowed to set conditions for all of or one of the targets and relation to be searched, and the stored binary relational information is searched while refining the binary relational information based on the set conditions. Therefore, it is possible to provide the knowledge search apparatus, the knowledge search method, the program, and the recording medium capable of efficiently searching for information on a desired target.

According to the present invention, one of the targets and the relation and the corresponding synonym are stored while making them correspond to each other, and the synonym corresponding to one of the searched targets and relation is searched. Therefore, it is possible to provide the knowledge search apparatus, the knowledge search method, the program, and the recording medium capable of expanding the search range up to synonyms and improving knowledge search accuracy.

According to the present invention, one of the targets and the relation and the corresponding hierarchical concept item are stored while making them correspond to each other, and all of or one of the targets and the relation included in the hierarchical concept item corresponding to one of the searched targets and relation is searched. Therefore, it is possible to provide the knowledge search apparatus, the knowledge search method, the program, and the recording medium capable of expanding the search range up to a generic concept or a subordinate concept and further improving knowledge search accuracy.

According to the present invention, the binary relational information is step-executed based on at least one of the number of searches, the number of searched targets, and hierarchies of searches. Therefore, it is possible to provide the knowledge search apparatus, the knowledge search method, the program, and the recording medium capable of executing the processings while the user check the processing results to some extent and selectively conducting a search in a necessary range.

### INDUSTRIAL APPLICABILITY

As explained so far, the knowledge search apparatus, the knowledge search method, the program, and the recording medium according to the present invention are employed for knowledge search such as data mining, and suited to the search of various pieces of knowledge from technical literatures, drug design and the like using search results for bioinformatics, cheminformatics, pharmainformatics, and the like.

## Claims

1. A knowledge search apparatus comprising:
a binary relational information storage unit that stores binary relational information while making a relational source target, a relational destination target, and a relation between the relational source target and the relational destination target correspond to one another;
a search condition setting unit that allows a user to set a.search start target and a search end target; and
a search unit that searches for all of or one of the targets and the relation for the search start target and the search end target from the binary relational information stored in the binary relational information storage unit, wherein
knowledge between the search start condition and the search end condition is automatically searched.

2. The knowledge search apparatus according to claim 1, further comprising:
a binary relational information creation unit that creates the binary relational information based on information on the targets and the relation expressed by a monadic relation or a polynomial relation of three terms or more.

3. The knowledge search apparatus according to claim 1 or 2, wherein the search unit further comprises:
a forward search unit that searches for the binary relational information stored in the binary relational information storage unit based on the relational source target.

4. The knowledge search apparatus according to claim 1 or 2, wherein the search unit further comprises:
a backward search unit that searches for the binary relational information stored in the binary relational information storage unit based on the relational destination target.

5. The knowledge search apparatus according to claim 1 or 2, wherein the search unit further comprises:
a target queue storage unit that sequentially stores the search targets in a queue;
a breadth-first search unit that searches for the binary relational information stored in the binary relational information storage unit in an order of storing the searched targets in the queue.

6. The knowledge search apparatus according to claim 5 wherein
the search unit further comprises:
a target overlap elimination unit that excludes overlap of the targets stored in the queue, wherein
the breadth-first search unit conducts a search for the queue from which the overlap of the targets is eliminated by the target overlap elimination unit.

7. The knowledge search apparatus according to claim 1 or 2, further comprising:
a condition setting unit that allows the user to set the conditions for all of or one of the targets and the relation to be searched, wherein
the search unit further comprises:
a condition search unit that searches the binary relational information stored in the binary relational information storage unit by refining the binary relational information based on the conditions set by the condition setting unit.

8. The knowledge search apparatus according to claim 1 or 2, further comprising:
a synonym storage unit that stores one of the targets and the relation searched and a corresponding synonym while making the synonym correspond to one of the targets and the relation, wherein
the search unit further comprises:
a synonym search unit that searches the synonym corresponding to one of the targets and the relation searched.

9. The knowledge search apparatus according to claim 1 or 2, further comprising:
an ontology storage unit that stores one of the targets and the relation searched and a corresponding hierarchical concept item the hierarchical concept item correspond to one of the targets and the relation, wherein
the search unit further comprises:
an ontology search unit that searches for all of or one of the targets and the relation included in the hierarchical concept item corresponding to one of the targets and the relation searched.

10. The knowledge search apparatus according to claim 1 or 2, further comprising:
a step-execution unit that step-executes a binary relation search by the search unit based on at leas one of the number of searches done by the search unit, the number of targets searches, and hierarchies of the searches.

11. A knowledge search method comprising:
a binary relational information storage step that stores binary relational information while making a relational source target, a relational destination target, and a relation between the relational source target and the relational destination target correspond to one another;
a search condition setting step that allows a user to set a search start target and a search end target; and
a search step that searches for all of or one of the targets and the relation for the search start target and the search end target from the binary relational information stored in the binary relational information storage step, wherein
knowledge between the search start condition and the search end condition is automatically searched.

12. The knowledge search method according to claim 11, further comprising:
a binary relational information creation step that creates the binary relational information based on information on the targets and the relation expressed by a monadic relation or a polynomial relation of three terms or more.

13. The knowledge search method according to claim 11 or 12, wherein
the search step further comprises:
a forward search step that searches for the binary relational information stored in the binary relational information storage step based on the relational source target.

14. The knowledge search method according to claim 11 or 12, wherein
the search step further comprises:
a backward search step that searches for the binary relational information stored in the binary relational information storage step based on the relational destination target.

15. The knowledge search method according to claim 11 or 12, wherein
the search step further comprises:
a target queue storage step that sequentially stores the search targets in a queue;
a breadth-first search step that searches for the binary relational information stored in the binary relational information storage step in an order of storing the searched targets in the queue.

16. The knowledge search method according to claim 15 wherein
the search step further comprises:
a target overlap elimination step that excludes overlap of the targets stored in the queue, wherein
the breadth-first search step conducts a search for the queue from which the overlap of the targets is eliminated by the target overlap elimination step.

17. The knowledge search method according to claim 11 or 12, further comprising:
a condition setting step that allows the user to set the conditions for all of or one of the targets and the relation to be searched, wherein
the search step further comprises:
a condition search step that searches the binary relational information stored in the binary relational information storage step by refining the binary relational information based on the conditions set by the condition setting step.

18. The knowledge search method according to claim 11 or 12, further comprising:
a synonym storage step that stores one of the targets and the relation searched and a corresponding synonym while making the synonym correspond to one of the targets and the relation, wherein
the search step further comprises:
a synonym search step that searches the synonym corresponding to one of the targets and the relation searched.

19. The knowledge search method according to claim 11 or 12, further comprising:
an ontology storage step that stores one of the targets and the relation searched and a corresponding hierarchical concept item the hierarchical concept item correspond to one of the targets and the relation, wherein
the search step further comprises:
an ontology search step that searches for all of or one of the targets and the relation included in the hierarchical concept item corresponding to one of the targets and the relation searched.

20. The knowledge search method according to claim 11 or 12, further comprising:
a step-execution step that step-executes a binary relation search by the search step based on at leas one of the number of searches done by the search step, the number of targets searches, and hierarchies of the searches.

21. A computer program that makes a computer to execute a knowledge search method comprising:
a binary relational information storage step that stores binary relational information while making a relational source target, a relational destination target, and a relation between the relational source target and the relational destination target correspond to one another;
a search condition setting step that allows a user to set a search start target and a search end target; and
a search step that searches for all of or one of the targets and the relation for the search start target and the search end target from the binary relational information stored in the binary relational information storage step, wherein
knowledge between the search start condition and the search end condition is automatically searched.

22. The computer program according to claim 21, further comprising:
a binary relational information creation step that creates the binary relational information based on information on the targets and the relation expressed by a monadic relation or a polynomial relation of three terms or more.

23. The computer program according to claim 21 or 22, wherein
the search step further comprises:
a forward search step that searches for the binary relational information stored in the binary relational information storage step based on the relational source target.

24. The computer program according to claim 21 or 22, wherein
the search step further comprises:
a backward search step that searches for the binary relational information stored in the binary relational information storage step based on the relational destination target.

25. The computer program according to claim 21 or 22, wherein
the search step further comprises:
a target queue storage step that sequentially stores the search targets in a queue;
a breadth-first search step that searches for the binary relational information stored in the binary relational information storage step in an order of storing the searched targets in the queue.

26. The computer program according to claim 25 wherein
the search step further comprises:
a target overlap elimination step that excludes overlap of the targets stored in the queue, wherein
the breadth-first search step conducts a search for the queue from which the overlap of the targets is eliminated by the target overlap elimination step.

27. The computer program according to claim 21 or 22, further comprising:
a condition setting step that allows the user to set the conditions for all of or one of the targets and the relation to be searched, wherein
the search step further comprises:
a condition search step that searches the binary relational information stored in the binary relational information storage step by refining the binary relational information based on the conditions set by the condition setting step.

28. The computer program according to claim 21 or 22, further comprising:
a synonym storage step that stores one of the targets and the relation searched and a corresponding synonym while making the synonym correspond to one of the targets and the relation, wherein
the search step further comprises:
a synonym search step that searches the synonym corresponding to one of the targets and the relation searched.

29. The computer program according to claim 21 or 22, further comprising:
an ontology storage step that stores one of the targets and the relation searched and a corresponding hierarchical concept item the hierarchical concept item correspond to one of the targets and the relation, wherein
the search step further comprises:
an ontology search step that searches for all of or one of the targets and the relation included in the hierarchical concept item corresponding to one of the targets and the relation searched.

30. The computer program according to claim 21 or 22, further comprising:
a step-execution step that step-executes a binary relation search by the search step based on at leas one of the number of searches done by the search step, the number of targets searches, and hierarchies of the searches.

31. A computer readable recording medium storing a computer program that makes a computer to execute a knowledge search method comprising:
a binary relational information storage step that stores binary relational information while making a relational source target, a relational destination target, and a relation between the relational source target and the relational destination target correspond to one another;
a search condition setting step that allows a user to set a search start target and a search end target; and
a search step that searches for all of or one of the targets and the relation for the search start target and the search end target from the binary relational information stored in the binary relational information storage step, wherein
knowledge between the search start condition and the search end condition is automatically searched.

32. The recording medium according to claim 31, further comprising:
a binary relational information creation step that creates the binary relational information based on information on the targets and the relation expressed by a monadic relation or a polynomial relation of three terms or more.

33. The recording medium according to claim 31 or 32, wherein
the search step further comprises:
a forward search step that searches for the binary relational information stored in the binary relational information storage step based on the relational source target.

34. The recording medium according to claim 31 or 32, wherein
the search step further comprises:
a backward search step that searches for the binary relational information stored in the binary relational information storage step based on the relational destination target.

35. The recording medium according to claim 31 or 32, wherein
the search step further comprises:
a target queue storage step that sequentially stores the search targets in a queue;
a breadth-first search step that searches for the binary relational information stored in the binary relational information storage step in an order of storing the searched targets in the queue.

36. The recording medium according to claim 35 wherein
the search step further comprises:
a target overlap elimination step that excludes overlap of the targets stored in the queue, wherein
the breadth-first search step conducts a search for the queue from which the overlap of the targets is eliminated by the target overlap elimination step.

37. The recording medium according to claim 31 or 32, further comprising:
a condition setting step that allows the user to set the conditions for all of or one of the targets and the relation to be searched, wherein
the search step further comprises:
a condition search step that searches the binary relational information stored in the binary relational information storage step by refining the binary relational information based on the conditions set by the condition setting step.

38. The recording medium according to claim 31 or 32, further comprising:
a synonym storage step that stores one of the targets and the relation searched and a corresponding synonym while making the synonym correspond to one of the targets and the relation, wherein
the search step further comprises:
a synonym search step that searches the synonym corresponding to one of the targets and the relation searched.

39. The recording medium according to claim 31 or 32, further comprising:
an ontology storage step that stores one of the targets and the relation searched and a corresponding hierarchical concept item the hierarchical concept item correspond to one of the targets and the relation, wherein
the search step further comprises:
an ontology search step that searches for all of or one of the targets and the relation included in the hierarchical concept item corresponding to one of the targets and the relation searched.

40. The recording medium according to claim 31 or 32, further comprising:
a step-execution step that step-executes a binary relation search by the search step based on at leas one of the number of searches done by the search step, the number of targets searches, and hierarchies of the searches.
